(19) Europäisches Patentamt
European Patent Office
Office européen des brevets

(11) Veröffentlichungsnummer: **0 372 326 A2**

(12) **EUROPÄISCHE PATENTANMELDUNG**

(21) Anmeldenummer: **89121778.8**

(22) Anmeldetag: **24.11.89**

(51) Int. Cl.⁵: **C07D 251/46, C07D 239/52, C07D 239/69, C07D 239/42, A01N 43/40, A01N 43/66**

(30) Priorität: **07.12.88 DE 3841086**

(43) Veröffentlichungstag der Anmeldung: **13.06.90 Patentblatt 90/24**

(84) Benannte Vertragsstaaten: **BE CH DE FR GB IT LI NL**

(71) Anmelder: **BAYER AG**

**D-5090 Leverkusen 1 Bayerwerk(DE)**

(72) Erfinder: **Gesing, Ernst R., Dr.**
**Trillser Graben 4**
**D-4006 Erkrath-Hochdahl(DE)**
Erfinder: **Santel, Hans-Joachim, Dr.**
**Grünstrasse 9a**
**D-5090 Leverkusen 1(DE)**
Erfinder: **Schmidt, Robert R., Dr.**
**Im Waldwinkel 110**
**D-5060 Bergisch Gladbach 2(DE)**
Erfinder: **Strang, Harry, Dr.**
**Heiderweg 53**
**D-4000 Düsseldorf 31(DE)**

(54) **Sulfonylaminoazine, Verfahren zu ihrer Herstellung und ihrer Verwendung als Herbizide.**

(57) Die Erfindung betrifft neue Sulfonylaminoazine der allgemeinen Formel (I)

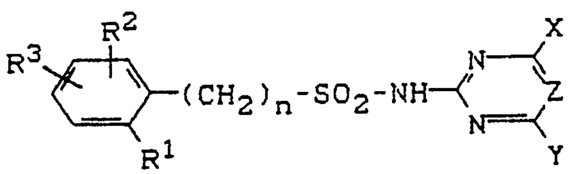

(I)

in welcher
$R^1$ für Halogen, Cyano, Nitro, $C_1$-$C_2$-Alkyl, $C_1$-$C_2$-Halogenalkyl, $C_1$-$C_2$-Alkoxy, $C_1$-$C_2$-Halogenalkoxy, $C_1$-$C_2$-Alkylthio, $C_1$-$C_2$-Halogenalkylthio, $C_1$-$C_2$-Alkylsulfinyl, $C_1$-$C_2$-Halogenalkylsulfinyl, $C_1$-$C_2$-Alkylsulfonyl, $C_1$-$C_2$-Halogenalkylsulfonyl, $C_1$-$C_2$-Alkoxy-$C_1$-$C_2$-alkoxy, Di-($C_1$-$C_2$-alkylamino)-sulfonyl, N-($C_1$-$C_2$-Alkoxy)-N-($C_1$-$C_2$-alkyl)-aminosulfonyl oder $C_1$-$C_2$-Alkoxy-carbonyl steht,
$R^2$ und $R^3$ unabhängig voneinander für Wasserstoff, Halogen, Alkyl oder Halogenalkyl stehen,
n für die Zahlen 0 oder 1 steht,
X für Wasserstoff, $C_1$-$C_2$-Alkyl, $C_1$-$C_2$-Halogenalkyl, $C_1$-$C_2$-Alkoxy-$C_1$-$C_2$-alkyl, Cyclopropyl, $C_1$-$C_2$-Alkoxy, $C_1$-$C_2$-Halogenalkoxy, $C_1$-$C_2$-Alkoxy-$C_1$-$C_2$-alkoxy, $C_1$-$C_2$-Alkylthio, $C_1$-$C_2$-Alkylamino oder Di-($C_1$-$C_2$-alkyl)-amino steht,
Y für Halogen, $C_1$-$C_2$-Alkyl, $C_1$-$C_2$-Halogenalkyl, Cyclopropyl, $C_1$-$C_2$-Alkoxy, $C_1$-$C_2$-Halogenalkoxy oder $C_1$-$C_2$-Alkylthio steht und
Z für Stickstoff oder eine CH-Gruppierung steht,
und Salze von Verbindungen der Formel (I), sowie Verfahren zu ihrer Herstellung und ihre Verwendung als Herbizide.

EP 0 372 326 A2

## Sulfonylaminoazine, Verfahren zu ihrer Herstellung und ihre Verwendung als Herbizide

Die Erfindung betrifft neue Sulfonylaminoazine, Verfahren zu ihrer Herstellung und ihre Verwendung als Herbizide.

Es wurden neue Sulfonylaminoazine der allgemeinen Formel (I)

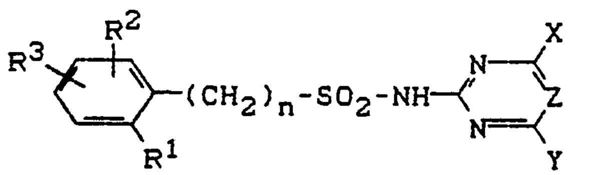

(I)

in welcher

$R^1$ für Halogen, Cyano, Nitro, $C_1$-$C_2$-Alkyl, $C_1$-$C_2$-Halogenalkyl, $C_1$-$C_2$-Alkoxy, $C_1$-$C_2$-Halogenalkoxy, $C_1$-$C_2$-Alkylthio, $C_1$-$C_2$-Halogenalkylthio, $C_1$-$C_2$-Alkylsulfinyl, $C_1$-$C_2$-Halogenalkylsulfinyl, $C_1$-$C_2$-Alkylsulfonyl, $C_1$-$C_2$-Halogenalkylsulfonyl, $C_1$-$C_2$-Alkoxy-$C_1$-$C_2$-alkoxy, Di-($C_1$-$C_2$-alkylamino)-sulfonyl, N-($C_1$-$C_2$-Alkoxy)-N-($C_1$-$C_2$-alkyl)-aminosulfonyl oder $C_1$-$C_2$-Alkoxy-carbonyl steht,

$R^2$ und $R^3$ unabhängig voneinander für Wasserstoff, Halogen, Alkyl oder Halogenalkyl stehen,

n für die Zahlen 0 oder 1 steht,

X für Wasserstoff, $C_1$-$C_2$-Alkyl, $C_1$-$C_2$-Halogenalkyl, $C_1$-$C_2$-Alkoxy-$C_1$-$C_2$-alkyl, Cyclopropyl, $C_1$-$C_2$-Alkoxy, $C_1$-$C_2$-Halogenalkoxy, $C_1$-$C_2$-Alkoxy-$C_1$-$C_2$-alkoxy, $C_1$-$C_2$-Alkylthio, $C_1$-$C_2$-Alkylamino oder Di-($C_1$-$C_2$-alkyl)-amino steht,

Y für Halogen, $C_1$-$C_2$-Alkyl, $C_1$-$C_2$-Halogenalkyl, Cyclopropyl, $C_1$-$C_2$-Alkoxy, $C_1$-$C_2$-Halogenalkoxy oder $C_1$-$C_2$-Alkylthio steht und

Z für Stickstoff oder eine CH-Gruppierung steht,

sowie Salze von Verbindungen der Formel (I)

gefunden.

Man erhält die neuen Sulfonylaminoazine der allgemeinen Formel (I), wenn man

(a) Sulfonsäureamide der allgemeinen Formel (II)

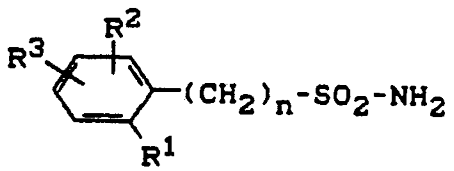

(II)

in welcher

$R^1$, $R^2$, $R^3$ und n die oben angegebenen Bedeutungen haben,

mit Azinen der allgemeinen Formel (III)

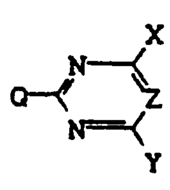

(III)

in welcher

X, Y und Z die oben angegebenen Bedeutungen haben und

Q für eine nucleophile Abgangsgruppe steht,

gegebenenfalls in Gegenwart eines Säureakzeptors und gegebenenfalls in Gegenwart eines Verdünnungsmittels umsetzt, oder wenn man

(b) Sulfonsäurechloride der allgemeinen Formel (IV)

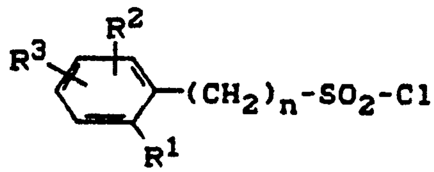

(IV)

in welcher
$R^1$, $R^2$, $R^3$ und n die oben angegebenen Bedeutungen haben,
mit Aminoazinen der allgemeinen Formel (V)

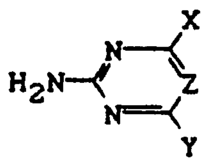

(V)

in welcher
X, Y und Z die oben angegebenen Bedeutungen haben,
gegebenenfalls in Gegenwart eines Säureakzeptors und gegebenenfalls in Gegenwart eines Verdünnungsmittels umsetzt.

Die neuen Sulfonylaminoazine der allgemeinen Formel (I) zeichnen sich durch starke herbizide Wirksamkeit aus.

Gegenstand der Erfindung sind vorzugsweise Verbindungen der Formel (I), in welcher
$R^1$ für Fluor, Chlor, Brom, Iod, Cyano, Nitro, Methyl, Trifluormethyl, Chlordifluormethyl, Difluormethyl, Methoxy, Ethoxy, Difluormethoxy, Trifluormethoxy, Chlordifluormethoxy, 2-Chlor-ethoxy 1,1,2,2-Tetrafluorethoxy 2-Chlor-1,1,2-trifluorethoxy, Methyl thio, Ethylthio, Difluormethylthio, Trifluormethylthio, Chlordifluormethylthio, Methylsulfinyl, Ethylsulfinyl, Trifluormethylsulfinyl, Methylsulfonyl, Ethylsulfonyl, Trifluormethylsulfonyl, 2-Methoxy-ethoxy, 2-Ethoxy-ethoxy, Dimethylaminosulfonyl, N-Methoxy-N-methylaminosulfonyl, Methoxycarbonyl oder Ethoxycarbonyl steht,
$R^2$ und $R^3$ unabhängig voneinander für Wasserstoff, Fluor, Chlor, Brom, Methyl oder Trifluormethyl stehen,
n für die Zahlen 0 oder 1 steht,
X für Wasserstoff, Methyl, Ethyl, Trifluormethyl, Chlormethyl, Methoxymethyl, Cyclopropyl, Methoxy, Ethoxy, Difluormethoxy, 2,2,2-Trifluorethoxy, Methoxymethoxy, 2-Methoxy-ethoxy, Methylthio, Methylamino, Ethylamino oder Dimethylamino steht,
Y für Fluor, Chlor, Brom, Methyl, Ethyl, Trifluormethyl, Chlormethyl, Cyclopropyl, Methoxy, Ethoxy, Difluormethoxy, 2,2,2-Trifluorethoxy oder Methylthio steht und
Z für Stickstoff oder eine CH-Gruppierung steht,
sowie die Natrium- und Kaliumsalze der Verbindungen der Formel (I).

Die Erfindung betrifft insbesondere Verbindungen der Formel (I), in welcher
$R^1$ für Fluor, Chlor, Brom, Methyl, Trifluormethyl, Difluormethoxy, Trifluormethoxy, 2-Chlor-ethoxy, Methylthio, Methylsulfonyl, Dimethylaminosulfonyl, Methoxycarbonyl oder Ethoxycarbonyl steht,
$R^2$ für Wasserstoff, Fluor oder Chlor steht,
$R^3$ für Wasserstoff steht,
n für die Zahlen 0 oder 1 steht,
X für Methyl, Methoxy oder Ethoxy steht,
Y für Methyl, Methoxy oder Ethoxy steht und
Z für Stickstoff oder eine CH-Gruppierung steht.

Beispiele für die erfindungsgemäßen Verbindungen der Formel (I) sind in der nachstehenden Tabelle 1 aufgeführt.

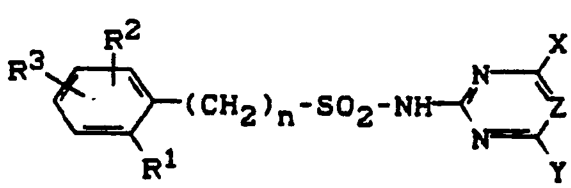

(I)

Tabelle 1: Beispiele für die Verbindungen der Formel (I)

| $R^1$ | $R^2$ | $R^3$ | n | X | Y | Z |
|---|---|---|---|---|---|---|
| F | H | H | 0 | $CH_3$ | $CH_3$ | CH |
| F | H | H | 0 | $CH_3$ | $CH_3$ | N |
| F | H | H | 0 | $CH_3$ | $OCH_3$ | CH |
| F | H | H | 0 | $CH_3$ | $OCH_3$ | N |
| F | H | H | 0 | $CH_3$ | $OC_2H_5$ | CH |
| F | H | H | 0 | $CH_3$ | $OC_2H_5$ | N |
| F | H | H | 0 | $OCH_3$ | $OCH_3$ | CH |
| F | H | H | 0 | $OCH_3$ | $OCH_3$ | N |
| F | H | H | 0 | $OC_2H_5$ | $OC_2H_5$ | N |
| Cl | H | H | 0 | $CH_3$ | $CH_3$ | CH |
| Cl | H | H | 0 | $CH_3$ | $CH_3$ | N |
| Cl | H | H | 0 | $CH_3$ | $OCH_3$ | CH |
| Cl | H | H | 0 | $CH_3$ | $OCH_3$ | N |
| Cl | H | H | 0 | $CH_3$ | $OC_2H_5$ | CH |
| Cl | H | H | 0 | $CH_3$ | $OC_2H_5$ | N |
| Cl | H | H | 0 | $OCH_3$ | $OCH_3$ | CH |
| Cl | H | H | 0 | $OCH_3$ | $OCH_3$ | N |
| Cl | H | H | 0 | $OC_2H_5$ | $OC_2H_5$ | N |
| Cl | H | H | 1 | $OCH_3$ | $OCH_3$ | CH |
| Cl | H | H | 1 | $OCH_3$ | $OCH_3$ | N |
| Cl | (2-)F | H | 0 | $CH_3$ | $CH_3$ | CH |

Tabelle 1 - Fortsetzung

| $R^1$ | $R^2$ | $R^3$ | n | X | Y | Z |
|---|---|---|---|---|---|---|
| Cl | (2-)Cl | H | 0 | $CH_3$ | $CH_3$ | CH |
| Br | H | H | 0 | $CH_3$ | $CH_3$ | CH |
| Br | H | H | 0 | $CH_3$ | $CH_3$ | N |
| Br | H | H | 0 | $CH_3$ | $OCH_3$ | CH |
| Br | H | H | 0 | $CH_3$ | $OCH_3$ | N |
| Br | H | H | 0 | $OCH_3$ | $OCH_3$ | CH |
| Br | H | H | 0 | $OCH_3$ | $OCH_3$ | N |
| Br | H | H | 0 | $OC_2H_5$ | $OC_2H_5$ | N |
| $CF_3$ | H | H | 0 | $CH_3$ | $CH_3$ | CH |
| $CF_3$ | H | H | 0 | $CH_3$ | $CH_3$ | N |
| $CF_3$ | H | H | 0 | $CH_3$ | $OCH_3$ | CH |
| $CF_3$ | H | H | 0 | $CH_3$ | $OCH_3$ | N |
| $CF_3$ | H | H | 0 | $OCH_3$ | $OCH_3$ | N |
| $CF_3$ | H | H | 0 | $OCH_3$ | $OCH_3$ | CH |
| $CF_3$ | H | H | 0 | $OC_2H_5$ | $OC_2H_5$ | N |
| $OCHF_2$ | H | H | 0 | $CH_3$ | $CH_3$ | CH |
| $OCHF_2$ | H | H | 0 | $CH_3$ | $CH_3$ | N |
| $OCHF_2$ | H | H | 0 | $CH_3$ | $OCH_3$ | CH |
| $OCHF_2$ | H | H | 0 | $CH_3$ | $OCH_3$ | N |
| $OCHF_2$ | H | H | 0 | $OCH_3$ | $OCH_3$ | CH |
| $OCHF_2$ | H | H | 0 | $OCH_3$ | $OCH_3$ | N |

Tabelle 1 - Fortsetzung

| $R^1$ | $R^2$ | $R^3$ | n | X | Y | Z |
|---|---|---|---|---|---|---|
| $OCHF_2$ | H | H | 0 | $OC_2H_5$ | $OC_2H_5$ | N |
| $OCF_3$ | H | H | 0 | $CH_3$ | $CH_3$ | CH |
| $OCF_3$ | H | H | 0 | $CH_3$ | $CH_3$ | N |
| $OCF_3$ | H | H | 0 | $CH_3$ | $OCH_3$ | CH |
| $OCF_3$ | H | H | 0 | $CH_3$ | $OCH_3$ | N |
| $OCF_3$ | H | H | 0 | $OCH_3$ | $OCH_3$ | CH |
| $OCF_3$ | H | H | 0 | $OCH_3$ | $OCH_3$ | N |
| $OCF_3$ | H | H | 0 | $OC_2H_5$ | $OC_2H_5$ | N |
| $OCH_2CH_2Cl$ | H | H | 0 | $CH_3$ | $OCH_3$ | CH |
| $OCH_2CH_2Cl$ | H | H | 0 | $CH_3$ | $OCH_3$ | N |
| $OCH_2CH_2Cl$ | H | H | 0 | $OCH_3$ | $OCH_3$ | CH |
| $OCH_2CH_2Cl$ | H | H | 0 | $OCH_3$ | $OCH_3$ | N |
| $SCH_3$ | H | H | 0 | $CH_3$ | $CH_3$ | CH |
| $SCH_3$ | H | H | 0 | $CH_3$ | $CH_3$ | N |
| $SCH_3$ | H | H | 0 | $CH_3$ | $OCH_3$ | CH |
| $SCH_3$ | H | H | 0 | $CH_3$ | $OCH_3$ | N |
| $SCH_3$ | H | H | 0 | $OCH_3$ | $OCH_3$ | N |
| $SO_2CH_3$ | H | H | 0 | $CH_3$ | $CH_3$ | CH |
| $SO_2CH_3$ | H | H | 0 | $CH_3$ | $CH_3$ | N |
| $SO_2CH_3$ | H | H | 0 | $CH_3$ | $OCH_3$ | CH |
| $SO_2CH_3$ | H | H | 0 | $CH_3$ | $OCH_3$ | N |

Tabelle 1 - Fortsetzung

| $R^1$ | $R^2$ | $R^3$ | n | X | Y | Z |
|---|---|---|---|---|---|---|
| $SO_2CH_3$ | H | H | 0 | $OCH_3$ | $OCH_3$ | N |
| $SO_2N(CH_3)_2$ | H | H | 0 | $CH_3$ | $CH_3$ | CH |
| $SO_2N(CH_3)_2$ | H | H | 0 | $CH_3$ | $CH_3$ | N |
| $SO_2N(CH_3)_2$ | H | H | 0 | $CH_3$ | $OCH_3$ | CH |
| $SO_2N(CH_3)_2$ | H | H | 0 | $CH_3$ | $OCH_3$ | N |
| $SO_2N(CH_3)_2$ | H | H | 0 | $OCH_3$ | $OCH_3$ | CH |
| $SO_2N(CH_3)_2$ | H | H | 0 | $OCH_3$ | $OCH_3$ | N |
| $SO_2N(CH_3)_2$ | H | H | 0 | $OC_2H_5$ | $OC_2H_5$ | N |
| $COOCH_3$ | H | H | 0 | $CH_3$ | $CH_3$ | CH |
| $COOCH_3$ | H | H | 0 | $CH_3$ | $CH_3$ | N |
| $COOCH_3$ | H | H | 0 | $CH_3$ | $OCH_3$ | CH |
| $COOCH_3$ | H | H | 0 | $CH_3$ | $OC_2H_5$ | CH |
| $COOCH_3$ | H | H | 0 | $CH_3$ | $OCH_3$ | N |
| $COOCH_3$ | H | H | 0 | $CH_3$ | $OC_2H_5$ | N |
| $COOCH_3$ | H | H | 0 | $OCH_3$ | $OCH_3$ | CH |
| $COOCH_3$ | H | H | 0 | $OCH_3$ | $OCH_3$ | N |
| $COOCH_3$ | H | H | 0 | $OC_2H_5$ | $OC_2H_5$ | CH |
| $COOCH_3$ | H | H | 0 | $OC_2H_5$ | $OC_2H_5$ | N |
| $COOC_2H_5$ | H | H | 0 | $CH_3$ | $CH_3$ | CH |
| $COOC_2H_5$ | H | H | 0 | $CH_3$ | $CH_3$ | N |
| $COOC_2H_5$ | H | H | 0 | $CH_3$ | $OCH_3$ | CH |

<u>Tabelle 1</u> - Fortsetzung

| $R^1$ | $R^2$ | $R^3$ | n | X | Y | Z |
|---|---|---|---|---|---|---|
| $COOC_2H_5$ | H | H | 0 | $CH_3$ | $OCH_3$ | N |
| $COOC_2H_5$ | H | H | 0 | $OCH_3$ | $OCH_3$ | CH |
| $COOC_2H_5$ | H | H | 0 | $OCH_3$ | $OCH_3$ | N |
| $COOCH_3$ | H | H | 1 | $OCH_3$ | $OCH_3$ | CH |
| $COOCH_3$ | H | H | 1 | $OCH_3$ | $OCH_3$ | N |
| $OCHF_2$ | H | H | 1 | $OCH_3$ | $OCH_3$ | CH |
| $OCHF_2$ | H | H | 1 | $OCH_3$ | $OCH_3$ | N |
| $OCF_3$ | H | H | 1 | $OCH_3$ | $OCH_3$ | CH |
| $OCF_3$ | H | H | 1 | $OCH_3$ | $OCH_3$ | N |
| $CH_3$ | (6-)Cl | H | 0 | $OCH_3$ | $OCH_3$ | N |

Verwendet man für das erfindungsgemäße Verfahren (a) beispielsweise 2-Fluor-benzolsulfonsäureamid und 2-Chlor-4,6-dimethoxy-s-triazin als Ausgangsstoffe so kann der Reaktionsablauf durch folgendes Formelschema skizziert werden:

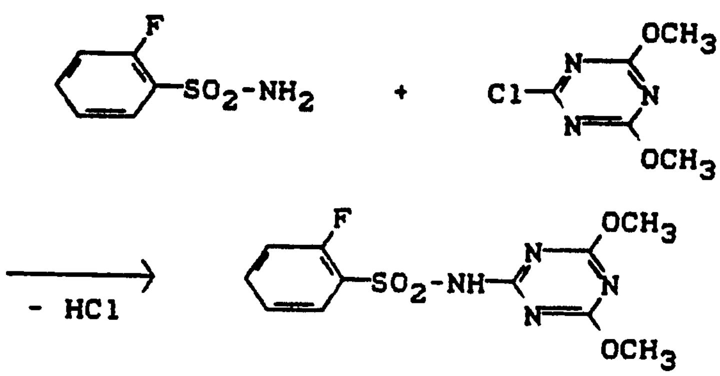


Verwendet man für das erfindungsgemäße Verfahren (b) beispielsweise 2-Chlor-benzolsulfonsäurechlorid und 2-Amino-4,6-dimethyl-pyrimidin als Ausgangsstoffe, so kann der Reaktionsablauf durch das folgende Formelschema skizziert werden:

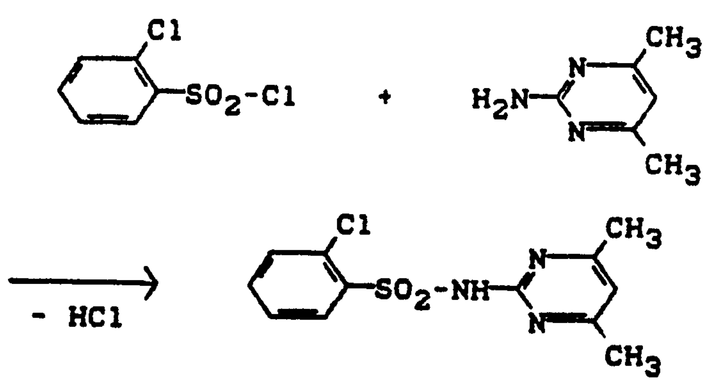

Die beim erfindungsgemäßen Verfahren (a) zur Herstellung von Verbindungen der Formel (I) als Ausgangsstoffe zu verwendenden Sulfonsäureamide sind durch die Formel (II) allgemein definiert.

In Formel (II) haben $R^1$, $R^2$, $R^3$ und n vorzugsweise bzw. insbesondere diejenigen Bedeutungen, die bereits oben im Zusammenhang mit der Beschreibung der erfindungsgemäßen Verbindungen der Formel (I) vorzugsweise bzw. als insbesondere bevorzugt für $R^1$, $R^2$, $R^3$ und n angegeben wurden.

Als Beispiele für die Ausgangsstoffe der Formel (II) seien genannt:
2-Fluor-, 2-Chlor-, 2-Brom-, 2,6-Dichlor-, 2-Chlor-6-fluor-, 2-Trifluormethyl-, 2-Difluormethoxy-, 2-Trifluormethoxy-, 2-(2-Chlor-ethoxy)-, 2-Methylthio-, 2-Methylsulfonyl-, 2-Dimethylaminosulfonyl-, 2-Methoxycarbonyl- und 2-Ethoxycarbonyl-benzolsulfonsäureamid sowie (2-Chlor-phenyl)-, (2-Difluormethoxy-phenyl)-, (2-Trifluormethoxy-phenyl)- und (2-Methoxycarbonyl-phenyl)-methansulfonsäureamid.

Die Ausgangsstoffe der Formel (II) sind bekannt und/oder können nach an sich bekannten Verfahren hergestellt werden (vgl. J. Org. Chem. 27 (1962), 1703 - 1709; US-P 4 371 391; US-P 4 310 346; US-P 4 452 628; EP-A 44 808; EP-A 87 780; US-P 4 732 711; EP-A 271 780).

Die beim erfindungsgemäßen Verfahren (a) weiter als Ausgangsstoffe zu verwendenden Azine sind durch die Formel (III) allgemein definiert.

In Formel (III) haben X, Y und Z vorzugsweise bzw. insbesondere diejenigen Bedeutungen, die bereits oben im Zusammenhang mit der Beschreibung der erfindungsgemäßen Verbindungen der Formel (I) vorzugsweise bzw. als insbesondere bevorzugt für X, Y und Z angegeben wurden und Q steht vorzugsweise für Fluor, Chlor, Methoxy, Ethoxy, Methylthio, Ethylthio, Benzylthio oder für die Gruppierung

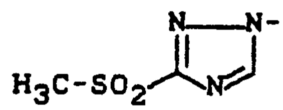

(3-Methylsulfonyl-1,2,4-triazol-1-yl); insbesondere die letztgenannte 3-Methylsulfonyl-1,2,4-triazol-1-yl-Gruppierung wird als Abgangsgruppe Q bevorzugt.

Als Beispiele für die Ausgangsstoffe der Formel (III) seien genannt:
2-(3-Methylsulfonyl-1,2,4-triazol-1-yl)-4,6-dimethyl-pyrimidin, -4-methoxy-6-methyl-pyrimidin, -4,6-dimethoxy-pyrimidin, -4-ethoxy-6-methyl-pyrimidin, -4,6-diethoxy-pyrimidin, -4,6-dimethyl-s-triazin, -4-methoxy-6-methyl-s-triazin, -4,6-dimethoxy-s-triazin, -4-ethoxy-6-methyl-s-triazin und -4,6-diethoxy-s-triazin.

Die Ausgangsstoffe der Formel (III) sind zum Teil bekannt (vgl. J. Chem. Soc. 1957, 1830, 1833; J. Org. Chem. 26 (1961), 792; US-P 3 308 119; US-P 4 711 959).

Neu sind die Ausgangsstoffe der Formel (IIIa)

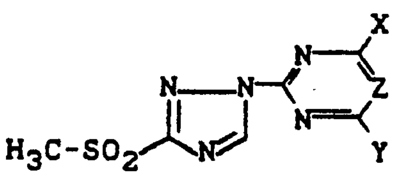

(IIIa)

in welcher
X, Y und Z die oben angegebenen Bedeutungen haben.

Man erhält die neuen 2-(3-Methylsulfonyl-1,2,4-triazol-1-yl)-azine der Formel (IIIa), wenn man Azine der Formel (IIIb)

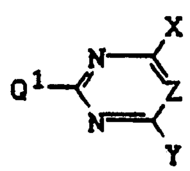

(IIIb)

in welcher
X, Y und Z die oben angegebenen Bedeutungen haben und
$Q^1$ für Chlor oder Methylsulfonyl steht,

mit 3-Methylthio-1,2,4-triazol der Formel (VI)

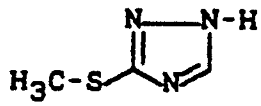

(VI)

gegebenenfalls in Gegenwart eines Säureakzeptors, wie z. B. Kaliumcarbonat und in Gegenwart eines Verdünnungs mittels, wie z. B. Acetonitril oder Dimethylformamid, bei Temperaturen zwischen 0 °C und 150 °C umsetzt und die hierbei erhaltenen 2-(3-Methylthio-1,2,4-triazol-1-yl)-azine der Formel (IIIc)

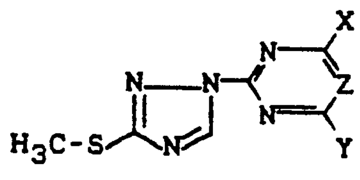

(IIIc)

mit einem Oxidationsmittel, wie z. B.

(a) Ameisensäure/Hydrogenperoxid, gegebenenfalls in Gegenwart eines Katalysators, wie z. B. Ammoniummolybdat, und in Gegenwart eines Verdünnungsmittels, wie z. B. Methylenchlorid und Wasser, oder

(b) Chlor in Gegenwart eines Verdünnungsmittels, wie z. B. Chloroform und Wasser, bei Temperaturen zwischen -20 °C und +50 °C umsetzt.

In den Formeln (IIIa), (IIIb) und (IIIc) haben X, Y und Z vorzugsweise bzw. insbesondere diejenigen Bedeutungen, die bereits oben im Zusammenhang mit der Beschreibung der erfindungsgemäßen Verbindungen der Formel (I) vorzugsweise bzw. als insbesondere bevorzugt für X, Y und Z angegeben wurden

Das erfindungsgemäße Verfahren (a) zur Herstellung der neuen Sulfonylaminoazine der Formel (I) wird vorzugsweise unter Verwendung von Verdünnungsmitteln durchgeführt.

Als Verdünnungsmittel kommen dabei praktisch alle inerten organischen Lösungsmittel in Frage. Hierzu gehören vorzugsweise aliphatische und aromatische, gegebenenfalls halogenierte Kohlenwasserstoffe wie Pentan, Hexan, Heptan, Cyclohexan, Petrolether, Benzin, Ligroin, Benzol, Toluol, Xylol, Methylenchlorid, Ethylenchlorid, Chloroform, Tetrachlorkohlenstoff, Chlorbenzol und o-Dichlorbenzol, Ether wie Diethyl- und Dibutylether, Glykoldimethylether und Diglykoldimethylether, Tetrahydrofuran und Dioxan, Ketone wie Aceton, Methyl-ethyl-, Methyl-isopropyl- und Methyl-isobutyl-keton, Ester wie Essigsäuremethylester und -ethylester, Nitrile wie z. B. Acetonitril und Propionitril, Amide wie z. B. Dimethylformamid, Dimethylacetamid und N-Methyl-pyrrolidon sowie Dimethylsulfoxid, Tetramethylensulfon und Hexamethylphosphorsäuretriamid.

Als Säureakzeptoren können bei dem erfindungsgemäßen Verfahren (a) alle üblicherweise für derartige Umsetzungen verwendbaren Säurebindemittel eingesetzt werden. Vorzugsweise in Frage kommen Alkalimetallhydroxide wie z. B. Natrium- und Kaliumhydroxid, Erdalkalihydroxide wie z. B. Calciumhydroxid, Alkalicarbonate und -alkoholate wie Natrium- und Kaliumcarbonat, Natrium- und Kalium-tert-butylat, ferner aliphatische, aromatische oder heterocyclische Amine, beispielsweise Triethylamin, Trimethylamin, Dimethylanilin, Dimethylbenzylamin, Pyridin, 1,5-Diazabicyclo-[4,3,0]-non-5-en (DBN), 1,8-Diazabicyclo-[5,4,0]-undec-7-en (DBU) und 1,4-Diazabicyclo-[2,2,2]-octan (DABCO).

Die Reaktionstemperaturen können bei dem erfindungsgemäßen Verfahren (a) in einem größeren Bereich variiert werden. Im allgemeinen arbeitet man bei Temperaturen zwischen 0 °C und 150 °C, vorzugsweise bei Temperaturen zwischen 20 °C und 100 °C.

Das erfindungsgemäße Verfahren (a) wird im allgemeinen unter Normaldruck durchgeführt. Es ist jedoch auch möglich, unter erhöhtem oder vermindertem Druck zu arbeiten.

Zur Durchführung des erfindungsgemäßen Verfahrens (a) werden die jeweils benötigten Ausgangsstoffe im allgemeinen in angenähert äquimolaren Mengen eingesetzt. Es ist jedoch auch möglich, eine der beiden jeweils eingesetzten Komponenten in einem größeren Überschuß zu verwenden. Die Reaktionen werden im allgemeinen in einem geeigneten Verdünnungsmittel in Gegenwart eines Säureakzeptors durchgeführt, und das Reaktionsgemisch wird mehrere Stunden bei der jeweils erforderlichen Temperatur gerührt. Die Aufarbeitung erfolgt bei dem erfindungsgemäßen Verfahren (a) jeweils nach üblichen Methoden.

Die beim erfindungsgemäßen Verfahren (b) zur Herstellung von Verbindungen der Formel (I) als

Ausgangsstoffe zu verwendenden Sulfonsäurechloride sind durch die Formel (IV) allgemein definiert.

In Formel (IV) haben $R^1$, $R^2$, $R^3$ und n vorzugsweise bzw. insbesondere diejenigen Bedeutungen, die bereits oben im Zusammenhang mit der Beschreibung der erfindungsgemäßen Verbindungen der Formel (I) vorzugsweise bzw. als insbesondere bevorzugt für $R^1$, $R^2$, $R^3$ und n angegeben wurden.

Als Beispiele für die Ausgangsstoffe der Formel (IV) seien genannt: 2-Fluor-, 2-Chlor-, 2-Brom-, 2,6-Dichlor-, 2-Chlor-6-fluor-, 2-Trifluormethyl-, 2-Difluormethoxy-, 2-Trifluormethoxy-, 2-(2-Chlor-ethoxy)-, 2-Methylthio-, 2-Methylsulfonyl-, 2-Dimethylaminosulfonyl-, 2-Methoxycarbonyl- und 2-Ethoxycarbonyl-benzolsulfonsäureamid sowie (2-Chlor-phenyl)-, (2-Difluormethoxy-phenyl)-, (2-Trifluormethoxy-phenyl)- und (2-Methoxycarbonyl-phenyl)-methansulfonsäurechlorid.

Die Ausgangsstoffe der Formel (IV) sind bekannt und/oder können nach an sich bekannten Verfahren hergestellt werden (vgl. J. Org. Chem. 33 (1968), 2104; J. Org. Chem. 25 (1960), 1824; DE-AS 23 08 262; EP-A 23 141; EP-A 23 422; EP-A 35 893; EP-A 48 143; EP-A 51 466; EP-A 64 322; EP-A 70 041; EP-A 44 808; US-P 2 929 820; US-P 4 282 242; US-P 4 348 220; US-P 4 372 778).

Die beim erfindungsgemäßen Verfahren (b) weiter als Ausgangsstoffe zu verwendenden Aminoazine sind durch die Formel (V) allgemein definiert.

In Formel (V) haben X, Y und Z vorzugsweise bzw. insbesondere diejenigen Bedeutungen, die bereits oben im Zusammenhang mit der Beschreibung der erfindungsgemäßen Verbindungen der Formel (I) vorzugsweise bzw. als insbesondere bevorzugt für X, Y und Z angegeben wurden.

Als Beispiele für die Ausgangsstoffe der Formel (V) seien genannt:
2-Amino-4,6-dimethyl-pyrimidin, -4-methoxy-6-methyl-pyrimidin, -4,6-dimethoxy-pyrimidin, -4-ethoxy-6-methyl-pyrimidin, -4,6-diethoxy-pyrimidin, -4,6-dimethyl-s-triazin, -4-methoxy-6-methyl-s-triazin, -4,6-dimethoxy-s-triazin, -4-ethoxy-6-methyl-s-triazin und -4,6-diethoxy-s-triazin.

Die Ausgangsstoffe der Formel (V) sind bekannt und/oder können nach an sich bekannten Verfahren hergestellt werden (vgl. Chem. Pharm. Bull. 11 (1963), 1382 - 1388; J. Chem. Soc. 1946, 81; US-P 4 299 960).

Das erfindungsgemäße Verfahren (b) wird vorzugsweise unter Verwendung von Verdünnungsmitteln durchgeführt. Es kommen insbesondere die gleichen Lösungsmittel in Betracht, wie sie oben für das erfindungsgemäße Verfahren (a) angegeben sind.

Als Säureakzeptoren können beim erfindungsgemäßen Verfahren (b) alle üblicherweise für derartige Umsetzungen verwendbaren Säurebindemittel eingesetzt werden. Es kommen vorzugsweise die gleichen Säurebindemittel in Betracht, wie sie oben für das erfindungsgemäße Verfahren (a) angegeben sind.

Die Reaktionstemperaturen können bei dem erfindungsge mäßen Verfahren (b) in einem größeren Bereich variiert werden. Im allgemeinen arbeitet man bei Temperaturen zwischen -30 °C und +80 °C, vorzugsweise bei Temperaturen zwischen 0 °C und 50 °C.

Das erfindungsgemäße Verfahren (b) wird im allgemeinen unter Normaldruck durchgeführt. Es ist jedoch auch möglich, unter erhöhtem oder vermindertem Druck zu arbeiten.

Zur Durchführung des erfindungsgemäßen Verfahrens (b) werden die jeweils benötigten Ausgangsstoffe im allgemeinen in angenähert äquimolaren Mengen eingesetzt. Es ist jedoch auch möglich, eine der beiden jeweils eingesetzten Komponenten in einem größeren Überschuß zu verwenden. Die Reaktionen werden im allgemeinen in einem geeigneten Verdünnungsmittel in Gegenwart eines Säureakzeptors durchgeführt, und das Reaktionsgemisch wird mehrere Stunden bei der jeweils erforderlichen Temperatur gerührt. Die Aufarbeitung erfolgt bei dem erfindungsgemäßen Verfahren (b) jeweils nach üblichen Methoden.

Die erfindungsgemäßen Wirkstoffe können als Defoliants, Desiccants, Krautabtötungsmittel und insbesondere als Unkrautvernichtungsmittel verwendet werden. Unter Unkraut im weitesten Sinne sind alle Pflanzen zu verstehen, die an Orten aufwachsen, wo sie unerwünscht sind. Ob die erfindungsgemäßen Stoffe als totale oder selektive Herbizide wirken, hängt im wesentlichen von der angewendeten Menge ab.

Die erfindungsgemäßen Wirkstoffe können z.B. bei den folgenden Pflanzen verwendet werden:

<u>Dikotyle Unkräuter der Gattungen:</u> Sinapis, Lepidium, Galium, Stellaria, Matricaria, Anthemis, Galinsoga, Chenopodium, Urtica, Senecio, Amaranthus, Portulaca, Xanthium, Convolvulus, Ipomoea, Polygonum, Sesbania, Ambrosia, Cirsium, Carduus, Sonchus, Solanum, Rorippa, Rotala, Lindernia, Lamium, Veronica, Abutilon, Emex, Datura, Viola, Galeopsis, Papaver, Centaurea.

<u>Dikotyle Kulturen der Gattungen:</u> Gossypium, Glycine, Beta, Daucus, Phaseolus, Pisum, Solanum, Linum, Ipomoea, Vicia, Nicotiana, Lycopersicon, Arachis, Brassica, Lactuca, Cucumis, Cucurbita.

<u>Monokotyle Unkräuter der Gattungen:</u> Echinochloa, Setaria, Panicum, Digitaria, Phleum, Poa, Festuca, Eleusine, Brachiaria, Lolium, Bromus, Avena, Cyperus, Sorghum, Agropyron, Cynodon, Monochoria, Fimbristylis, Sagittaria, Eleocharis, Scirpus, Paspalum, Ischaemum, Sphenoclea, Dactyloctenium, Agrostis, Alopecurus, Apera.

<u>Monokotyle Kulturen der Gattungen:</u> Oryza, Zea, Triticum, Hordeum, Avena, Secale, Sorghum, Panicum,

Saccharum, Ananas, Asparagus, Allium.

Die Verwendung der erfindungsgemäßen Wirkstoffe ist jedoch keineswegs auf diese Gattungen beschränkt, sondern erstreckt sich in gleicher Weise auch auf andere Pflanzen.

Die Verbindungen eignen sich in Abhängigkeit von der Konzentration zur Totalunkrautbekämpfung z.B. auf Industrie- und Gleisanlagen und auf Wegen und Plätzen mit und ohne Baumbewuchs. Ebenso können die Verbindungen zur Unkrautbekämpfung in Dauerkulturen, z.B. Forst, Ziergehölz-, Obst-, Wein-, Citrus-, Nuß-, Bananen-, Kaffee-, Tee-, Gummi-, Ölpalm-, Kakao-, Beerenfrucht- und Hopfenanlagen und zur selektiven Unkrautbekämpfung in einjährigen Kulturen eingesetzt werden.

Die erfindungsgemäßen Verbindungen der Formel (I) eignen sich insbesondere zur selektiven Bekämpfung von dikotylen Unkräutern in monokotylen Kulturen, vor allem im Nachauflauf-Verfahren.

Die Wirkstoffe können in die üblichen Formulierungen überführt werden, wie Lösungen, Emulsionen, Spritzpulver, Suspensionen, Pulver, Stäubemittel, Pasten, lösliche Pulver, Granulate, Suspensions-Emulsions-Konzentrate, Wirkstoff-imprägnierte Natur- und synthetische Stoffe sowie Feinstverkapselungen in polymeren Stoffen.

Diese Formulierungen werden in bekannter Weise hergestellt, z. B. durch Vermischen der Wirkstoffe mit Streckmitteln, also flüssigen Lösungsmitteln und/oder festen Trägerstoffen, gegebenenfalls unter Verwendung von oberflächenaktiven Mitteln, also Emulgiermitteln und/oder Dispergiermitteln und/oder schaumerzeugenden Mitteln.

Im Falle der Benutzung von Wasser als Streckmittel kön nen z.B. auch organische Lösungsmittel als Hilfslösungsmittel verwendet werden. Als flüssige Lösungsmittel kommen im wesentlichen in Frage: Aromaten, wie Xylol, Toluol, oder Alkylnaphthaline, chlorierte Aromaten und chlorierte aliphatische Kohlenwasserstoffe, wie Chlorbenzole, Chlorethylene oder Methylenchlorid, aliphatische Kohlenwasserstoffe, wie Cyclohexan oder Paraffine, z.B. Erdölfraktionen, mineralische und pflanzliche Öle, Alkohole, wie Butanol oder Glykol sowie deren Ether und Ester, Ketone wie Aceton, Methylethylketon, Methylisobutylketon oder Cyclohexanon, stark polare Lösungsmittel, wie Dimethylformamid und Dimethylsulfoxid, sowie Wasser.

Als feste Trägerstoffe kommen in Frage:

z.B. Ammoniumsalze und natürliche Gesteinsmehle, wie Kaoline, Tonerden, Talkum, Kreide, Quarz, Attapulgit, Montmorillonit oder Diatomeenerde und synthetische Gesteinsmehle, wie hochdisperse Kieselsäure, Aluminiumoxid und Silikate, als feste Trägerstoffe für Granulate kommen in Frage: z.B. gebrochene und fraktionierte natürliche Gesteine wie Calcit, Marmor, Bims, Sepiolith, Dolomit sowie synthetische Granulate aus anorganischen und organischen Mehlen sowie Granulate aus organischem Material wie Sägemehl, Kokosnußschalen, Maiskolben und Tabakstengeln; als Emulgier- und/oder schaumerzeugende Mittel kommen in Frage: z.B. nichtionogene und anionische Emulgatoren, wie Polyoxyethylen-Fettsäure-Ester, Polyoxyethylen-Fettalkohol-Ether, z.B. Alkylaryl-polyglykolether, Alkylsulfonate, Alkylsulfate, Arylsulfonate sowie Eiweißhydrolysate; als Dispergiermittel kommen in Frage:

z.B. Lignin-Sulfitablaugen und Methylcellulose.

Es können in den Formulierungen Haftmittel wie Carboxymethylcellulose, natürliche und synthetische pulvrige, körnige oder latexförmige Polymere verwendet werden, wie Gummiarabicum, Polyvinylalkohol, Polyvinylacetat, sowie natürliche Phospholipide, wie Kephaline und Lecithine und synthetische Phospholipide. Weitere Additive können mineralische und vegetabile Öle sein.

Es können Farbstoffe wie anorganische Pigmente, z.B. Eisenoxid, Titanoxid, Ferrocyanblau und organische Farbstoffe, wie Alizarin-, Azo- und Metallphthalocyaninfarbstoffe und Spurennährstoffe wie Salze von Eisen, Mangan, Bor, Kupfer, Kobalt, Molybdän und Zink verwendet werden.

Die Formulierungen enthalten im allgemeinen zwischen 0,1 und 95 Gewichtsprozent Wirkstoff, vorzugsweise zwischen 0,5 und 90 %.

Die erfindungsgemäßen Wirkstoffe können als solche oder in ihren Formulierungen auch in Mischung mit bekannten Herbiziden zur Unkrautbekämpfung Verwendung finden, wobei Fertigformulierungen oder Tankmischungen möglich sind.

Für die Mischungen kommen bekannte Herbizide wie z.B. 1-Amino-6-ethylthio-3-(2,2-dimethylpropyl)-1,3,5-triazin-2,4(1H,3H)-dion (AMETHYDIONE) oder N-(2-Benzthiazolyl)-N,N′-dimethyl-harnstoff (METABENZTHIAZURON) zur Unkrautbekämpfung in Getreide; 4-Amino-3-methyl-6-phenyl-1,2,4-triazin-5-(4H)-on (METAMITRON) zur Unkrautbekämpfung in Zuckerrüben und 4-Amino-6-(1,1-dimethylethyl)-3-methylthio-1,2,4-triazin-5(4H)-on (METRIBUZIN) zur Unkrautbekämpfung in Sojabohnen, in Frage; ferner auch 2,4-Dichlorphenoxyessigsäure (2,4-D); 4-(2,4-Dichlorphenoxy)-buttersäure (2,4-DB); 2,4-Dichlorphenoxypropionsäure (2,4-DP); 2-Chlor-4-ethylamino-6-isopropylamino-1,3,5-triazin (ATRAZIN); 3-Isopropyl-2,1,3-benzothiadiazin-4-on-2,2-dioxid (BENTAZON); Methyl-5-(2,4-dichlorphenoxy)-2-nitrobenzoat (BIFENOX); 3,5-Dibrom-4-hydroxy-benzonitril (BROMOXYNIL); 2-Chlor-N-{[(4-methoxy-6-methyl-1,3,5-triazin-2-yl)-amino]-carbonyl}-benzolsulfonamid (CHLORSULFURON); N,N-Dimethyl-N′-(3-chlor-4-methylphenyl)-harnstoff

(CHLORTOLURON); 2-Chlor-4-ethylamino-6-(3-cyanopropylamino)-1,3,5-triazin (CYANAZIN); 2-[4-(2,4-Dichlorphenoxy)-phenoxy]-propionsäure, deren Methyl- oder deren Ethylester (DICLOFOP); 4-Amino-6-t-butyl-3-ethylthio-1,2,4-triazin-5(4H)-on (ETHIOZIN); 2-{4-[(6-Chlor-2-benzoxazolyl)-oxy]-phenoxy}-propansäure, deren Methyl- oder deren Ethylester (FENOXAPROP); [(4-Amino-3,5-dichlor-6-fluor-2-pyridinyl)-oxy]-essigsäure bzw. deren 1-Methylheptylester (FLUROXYPYR); Methyl-2-[4,5-dihydro-4-methyl-4-(1-methylethyl)-5-oxo-1H-imidazol-2-yl]-4(5)-methylbenzoat (IMAZAMETHABENZ); 3,5-Diiod-4-hydroxybenzonitril (IOXYNIL); N,N-Dimethyl-N′-(4-isopropylphenyl)-harnstoff (ISOPROTURON); (2-Methyl-4-chlorphenoxy)-essigsäure (MCPA); (4-Chlor-2-methylphenoxy)-propionsäure (MCPP); N-Methyl-2-(1,3-benzthiazol-2-yloxy)-acetanilid (MEFENACET); 2-{[[((4-Methoxy-6-methyl-1,3,5-triazin-2-yl)-amino)-carbonyl]-amino]-sulfonyl}-benzoesäure oder deren Methylester (METSULFURON); N-(1-Ethylpropyl)-3,4-dimethyl-2,6-dinitroanilin (PENDIMETHALIN); 0-(6-Chlor-3-phenyl-pyridazin-4-yl)-S-octyl-thiocarbonat (PYRIDATE); 4-Ethylamino-2-t-butylamino-6-methylthio-s-triazin (TERBUTRYNE); 3-[[[[(4-Methoxy-6-methyl-1,3,5-triazin-2-yl)-amino]-carbonyl]-amino]-sulfonyl]-thiophen-2-carbonsäure-methylester (THIACARBURON) und 3-[[[[(4-Methoxy-6-methyl-1,3,5-triazin-2-yl)-amino]-carbonyl]-amino]-sulfonyl]-thiophen-2-carbonsäuremethylester (THIAMETURON). Einige Mischungen zeigen überraschenderweise auch synergistische Wirkung.

Auch eine Mischung mit anderen bekannten Wirkstoffen, wie Fungiziden, Insektiziden, Akariziden, Nematiziden, Schutzstoffen gegen Vogelfraß, Pflanzennährstoffen und Bodenstrukturverbesserungsmitteln ist möglich.

Die Wirkstoffe können als solche, in Form ihrer Formulierungen oder den daraus durch weiteres Verdünnen bereiteten Anwendungsformen, wie gebrauchsfertige Lösungen, Suspensionen, Emulsionen, Pulver, Pasten und Granulate angewandt werden. Die Anwendung geschieht in üblicher Weise, z.B. durch Gießen, Spritzen, Sprühen, Streuen.

Die erfindungsgemäßen Wirkstoffe können sowohl vor als auch nach dem Auflaufen der Pflanzen appliziert werden.

Sie können auch vor der Saat in den Boden eingearbeitet werden.

Die angewandte Wirkstoffmenge kann in einem größeren Bereich schwanken. Sie hängt im wesentlichen von der Art des gewünschten Effektes ab. Im allgemeinen liegen die Aufwandmengen zwischen 0,01 und 15 kg Wirkstoff pro Hektar Bodenfläche, vorzugsweise zwischen 0,05 und 10 kg pro ha.

Die Herstellung und die Verwendung der erfindungsgemäßen Wirkstoffe geht aus den nachfolgenden Beispielen hervor.

Herstellungsbeispiele

Beispiel 1

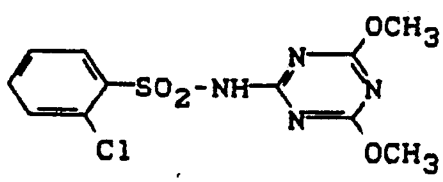

(Verfahren (a))

Eine Mischung aus 1,43 g (5 mMol) 2-(3-Methylsulfonyl-1,2,4-triazol-1-yl)-4,6-dimethoxy-s-triazin, 0,96 g (5 mMol) 2-Chlor-benzolsulfonsäureamid, 1,38 g (10 mMol) Kaliumcarbonat und 30 ml Acetonitril wird 12 Stunden unter Rückfluß zum Sieden erhitzt. Anschließend wird eingeengt, der Rückstand in 100 ml Wasser aufgenommen und mit konz. Salzsäure pH 1,5 eingestellt. Es wird dreimal mit je 50 ml Methylenchlorid extrahiert, die vereinigten Extraktionslösungen mit Natriumsulfat getrocknet und filtriert. Vom Filtrat wird das Lösungsmittel im Wasserstrahlvakuum sorgfältig abdestilliert.

Man erhält 0,93 g (57 % der Theorie) 2-(2-Chlor-phenylsulfonylamino)-4,6-dimethoxy-s-triazin als kristallinen Rückstand vom Schmelzpunkt 137 °C.

Analog Beispiel 1 und entsprechend der allgemeinen Beschreibung der erfindungsgemäßen Herstellungsverfahren können die in der nachstehenden Tabelle 2 aufgeführten Verbindungen der Formel (I)

hergestellt werden.

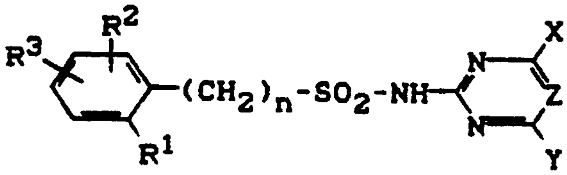

(I)

Tabelle 2: Beispiele für die Verbindungen der Formel (I)

| Beisp.-Nr. | $R^1$ | $R^2$ | $R^3$ | n | X | Y | Z | Physikalische Daten |
|---|---|---|---|---|---|---|---|---|
| 2 | $CH_3$ | (6-)Cl | H | 0 | $OCH_3$ | $OCH_3$ | N | Fp. 182 °C |
| 3 | $COOCH_3$ | H | H | 0 | $OCH_3$ | $OCH_3$ | N | Fp. 150 °C |
| 4 | F | H | H | 0 | $OCH_3$ | $OCH_3$ | N | Fp. 150 °C |
| 5 | Cl | H | H | 0 | $OC_2H_5$ | $OC_2H_5$ | N | Fp. 114 °C |
| 6 | Br | H | H | 0 | $OC_2H_5$ | $OC_2H_5$ | N | Fp. 134 °C |
| 7 | F | H | H | 0 | $OC_2H_5$ | $OC_2H_5$ | N | Fp. 112 °C |
| 8 | $CO_2CH_3$ | H | H | 0 | $OC_2H_5$ | $OC_2H_5$ | N | Fp. 139 °C |
| 9 | $SO_2N(CH_3)_2$ | H | H | 0 | $OC_2H_5$ | $OC_2H_5$ | N | Fp. 124 °C |
| 10 | $CF_3$ | H | H | 0 | $OC_2H_5$ | $OC_2H_5$ | N | Fp. 153 °C |
| 11 | $CH_3$ | (6-)Cl | H | 0 | $OC_2H_5$ | $OC_2H_5$ | N | Fp. 126 °C |
| 12 | $OCHF_2$ | H | H | 0 | $OC_2H_5$ | $OC_2H_5$ | N | Fp. 148 °C |
| 13 | Cl | (6-)Cl | H | 0 | $OC_2H_5$ | $OC_2H_5$ | N | Fp. 165 °C |

Fp. = Schmelzpunkt

<u>Tabelle 2</u> - Fortsetzung

| Bsp.-Nr. | $R^1$ | $R^2$ | $R^3$ | n | X | Y | Z | Physikalische Daten |
|---|---|---|---|---|---|---|---|---|
| 14 | Br | H | H | 0 | $OCH_3$ | $OCH_3$ | CH | Fp. 75° C |
| 15 | F | H | H | 0 | $CH_3$ | $CH_3$ | CH | Fp. 248° C *) |
| 16 | Cl | H | H | 0 | $CH_3$ | $CH_3$ | CH | Fp. 180° C *) |
| 17 | Br | H | H | 0 | $CH_3$ | $CH_3$ | CH | Fp. 175° C *) |
| 18 | $OCHF_2$ | H | H | 0 | $CH_3$ | $CH_3$ | CH | Fp. 247° C *) |
| 19 | Cl | (6-)Cl | H | 0 | $CH_3$ | $CH_3$ | CH | Fp. >250° C *) |
| 20 | $SO_2N(CH_3)_2$ | H | H | 0 | $CH_3$ | $CH_3$ | CH | Fp. 173° C *) |
| 21 | $COOCH_3$ | H | H | 0 | $CH_3$ | $CH_3$ | CH | Fp. 155° C *) |
| 22 | Cl | H | H | 0 | H | $CH_3$ | CH | Fp. 209° C *) |
| 23 | F | H | H | 0 | $CH_3$ | $CH_3$ | CH | Fp. 183° C |
| 24 | Cl | H | H | 0 | $CH_3$ | $CH_3$ | CH | Fp. 217° C |
| 25 | Br | H | H | 0 | $CH_3$ | $CH_3$ | CH | Fp. 211° C |
| 26 | $OCF_3$ | H | H | 0 | $CH_3$ | $CH_3$ | CH | Fp. 174° C |

*) Natriumsalz

<u>Tabelle 2</u> - Fortsetzung

| Bsp.-Nr. | $R^1$ | $R^2$ | $R^3$ | n | X | Y | Z | Physikalische Daten |
|---|---|---|---|---|---|---|---|---|
| 27 | $OCHF_2$ | H | H | 0 | $CH_3$ | $CH_3$ | CH | Fp. 162° C |
| 28 | Cl | (6-)Cl | H | 0 | $CH_3$ | $CH_3$ | CH | Fp. >250° C |
| 29 | $SO_2N(CH_3)_2$ | H | H | 0 | $CH_3$ | $CH_3$ | CH | Fp. 163° C |
| 30 | Cl | (6-)Cl | H | 1 | $CH_3$ | $CH_3$ | CH | Fp. 206° C |
| 31 | $COOCH_3$ | H | H | 0 | $CH_3$ | $CH_3$ | CH | Fp. 137° C |
| 32 | F | H | H | 0 | H | $CH_3$ | CH | Fp. 228° C |
| 33 | Cl | H | H | 0 | H | $CH_3$ | CH | Fp. >250° C |
| 34 | Br | H | H | 0 | H | $CH_3$ | CH | Fp. >250° C |
| 35 | $OCF_3$ | H | H | 0 | H | $CH_3$ | CH | Fp. 241° C |
| 36 | Cl | H | H | 0 | $CH_3$ | $OCH_3$ | CH | Fp. 163° C |
| 37 | $COOCH_3$ | H | H | 1 | $CH_3$ | $CH_3$ | CH | Fp. 164° C |
| 38 | Cl | H | H | 1 | $CH_3$ | $CH_3$ | CH | Fp. 163° C |
| 39 | $OCF_3$ | H | H | 1 | $CH_3$ | $CH_3$ | CH | Fp. 134° C |
| 40 | $COOC_2H_5$ | H | H | 0 | $CH_3$ | $CH_3$ | CH | Fp. 97° C |
| 41 | $CH_3$ | (6-)Cl | H | 0 | $CH_3$ | $CH_3$ | CH | Fp. >250° C |

Tabelle 2 - Fortsetzung

| Bsp.-Nr. | $R^1$ | $R^2$ | $R^3$ | n | X | Y | Z | Physikalische Daten |
|---|---|---|---|---|---|---|---|---|
| 42 | Cl | (4-)Cl | H | 0 | $CH_3$ | $CH_3$ | CH | Fp. >250° C |
| 43 | $SO_2N(CH_3)_2$ | H | H | 0 | H | $CH_3$ | CH | Fp. 188° C |
| 44 | $CH_3$ | (6-)Cl | H | 0 | H | $CH_3$ | CH | Fp. >250° C |
| 45 | $COOCH_3$ | H | H | 1 | H | $CH_3$ | CH | Fp. 164° C |

Ausgangsstoffe der Formel (IIIa)

Beispiel (IIIa-1)

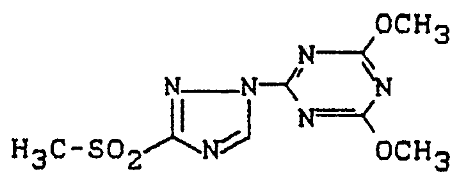

6,6 g 30 %ige Hydrogenperoxidlösung (in Wasser) (0,058 Mol) werden bei 15 °C bis 30 °C unter Rühren tropfenweise zu einer Mischung aus 3,8 g (0,015 Mol) 2-(3-Methylthio-1,2,4-triazol-1-yl)-4,6-dimethoxy-s-triazin, 1,75 g (0,038 Mol) Ameisensäure, 0,1 g Ammoniummolybdat-tetrahydrat und 30 ml Methylenchlorid gegeben. Das Reaktionsgemisch wird 15 Stunden bei 20 °C gerührt, dann mit Methylenchlorid und Wasser vedünnt und geschüttelt. Die organische Phase wird abgetrennt, mit Natriumhydrogensulfit-Lösung, dann mit Kaliumcarbonat-Lösung und schließlich mit Wasser gewaschen, mit Natriumsulfat getrocknet und filtriert. Vom Filtrat wird das Lösungsmittel im Wasserstrahlvakuum sorgfältig abdestilliert.

Man erhält 2,8 g (67 % der Theorie) 2-(3-Methylsulfonyl-1,2,4-triazol-1-yl)-4,6-dimethoxy-s-triazin als kristallinen Rückstand vom Schmelzpunkt 170 °C.

Beispiel (IIIa-2)

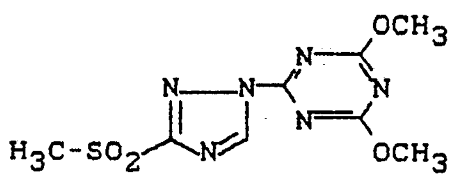

In eine Lösung von 3,8 g (0,015 Mol) 2-(3-Methylthio-1,2,4-triazol-1-yl)-4,6-dimethoxy-s-triazin in 25 ml Chloroform und 12,5 ml Wasser wird bei 0 °C bis 5 °C Chlorgas bis zur Sättigung eingeleitet und das Gemisch wird dann noch 10 Minuten gerührt, anschließend mit 100 ml Chloroform verdünnt und mit Wasser geschüttelt. Die organische Phase wird abgetrennt mit Natriumsulfat getrocknet und filtriert. Vom Filtrat wird das Lösungsmittel im Wasserstrahlvakuum abdestilliert.

Man erhält 2,8 g (67 % der Theorie) 2-(3-Methylsulfonyl-1,2,4-triazol-1-yl)-4,6-dimethoxy-s-triazin als kristallinen Rückstand vom Schmelzpunkt 170 °C.

Analog erhält man:

(IIIa-3)

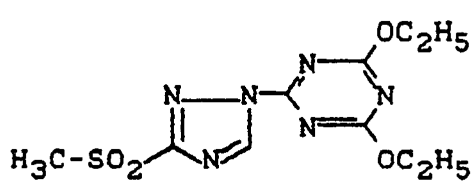

Schmelzpunkt: 98 °C

Ausgangsstoffe der Formel (IIIc)

Beispiel (IIIc-1)

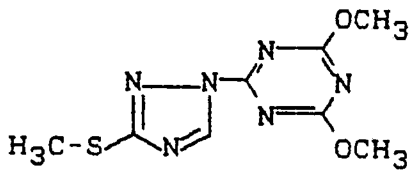

Eine Mischung aus 8,75 g (0,05 Mol) 2-Chlor-4,6-dimethoxy-s-triazin, 9,8 g (0,85 Mol) 3-Methylthio-1,2,4-triazol, 6,9 g (0,05 Mol) Kaliumcarbonat und 100 ml Acetonitril wird 20 Stunden unter Rückfluß zum Sieden erhitzt. Anschließend wird eingeengt, der Rückstand in 200 ml Methylenchlorid aufgenommen und zweimal mit je 100 ml Wasser gewaschen. Die organische Phase wird mit Natriumsulfat getrocknet und filtriert. Vom Filtrat wird das Lösungsmittel im Wasserstrahlvakuum abdestilliert.

Man erhält 11,7 g (92 % der Theorie) 2-(3-Methylthio-1,2,4-triazol-1-yl)-4,6-dimethoxy-s-triazin als kristallinen Rückstand vom Schmelzpunkt 160 °C.

Analog erhält man:

(IIIc-2)

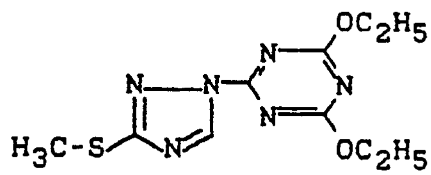

Schmelzpunkt: 157 °C

(IIIc-3)

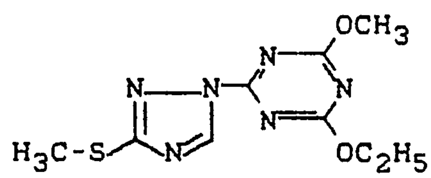

Schmelzpunkt: 155 °C

Anwendungsbeispiele

Beispiel A

Post-emergence-Test

Lösungsmittel: 5 Gewichtsteile Aceton
Emulgator: 1 Gewichtsteil Alkylarylpolyglykolether

Zur Herstellung einer zweckmäßigen Wirkstoffzubereitung vermischt man 1 Gewichtsteil Wirkstoff mit der angegebenen Menge Lösungsmittel, gibt die angegebene Menge Emulgator zu und verdünnt das Konzentrat mit Wasser auf die gewünschte Konzentration.

Mit der Wirkstoffzubereitung spritzt man Testpflanzen, welche eine Höhe von 5 - 15 cm haben so, daß die jeweils gewünschten Wirkstoffmengen pro Flächeneinheit ausgebracht werden. Die Konzentration der Spritzbrühe wird so gewählt, daß in 2000 l Wasser/ha die jeweils gewünschten Wirkstoffmengen ausgebracht werden. Nach drei Wochen wird der Schädigungsgrad der Pflanzen bonitiert in % Schädigung im Vergleich zur Entwicklung der unbehandelten Kontrolle.

Es bedeuten:
0 % = keine Wirkung (wie unbehandelte Kontrolle)
100 % = totale Vernichtung

In diesem Test zeigen z. B. die Verbindungen gemäß Herstellungsbeispielen 1, 4, 8 und 12 bei guter Selektivität in Weizen und Mais starke Wirkung gegen Unkräuter, wie z. B. Datura, Galium, Polygonum, Sinapis, Veronica, Helianthus und Xanthium.

## Ansprüche

1. Sulfonylaminoazine der allgemeinen Formel (I)

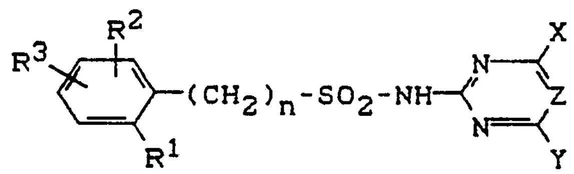

(I)

in welcher
$R^1$ für Halogen, Cyano, Nitro, $C_1$-$C_2$-Alkyl, $C_1$-$C_2$-Halogenalkyl, $C_1$-$C_2$-Alkoxy, $C_1$-$C_2$-Halogenalkoxy, $C_1$-$C_2$-Alkylthio, $C_1$-$C_2$-Halogenalkylthio $C_1$-$C_2$-Alkylsulfinyl, $C_1$-$C_2$-Halogenalkylsulfinyl, $C_1$-$C_2$-Alkylsulfonyl, $C_1$-$C_2$-Halogenalkylsulfonyl, $C_1$-$C_2$-Alkoxy-$C_1$-$C_2$-alkoxy, Di-($C_1$-$C_2$-alkylamino)-sulfonyl, N-($C_1$-$C_2$-Alkoxy)-N-($C_1$-$C_2$-alkyl)-aminosulfonyl oder $C_1$-$C_2$-Alkoxy-carbonyl steht,
$R^2$ und $R^3$ unabhängig voneinander für Wasserstoff, Halogen, Alkyl oder Halogenalkyl stellen,
n für die Zahlen 0 oder 1 steht,
X für Wasserstoff, $C_1$-$C_2$-Alkyl, $C_1$-$C_2$-Halogenalkyl, $C_1$-$C_2$-Alkoxy-$C_1$-$C_2$-alkyl, Cyclopropyl, $C_1$-$C_2$-Alkoxy, $C_1$-$C_2$-Halogenalkoxy, $C_1$-$C_2$-Alkoxy-$C_1$-$C_2$-alkoxy, $C_1$-$C_2$-Alkylthio, $C_1$-$C_2$-Alkylamino oder Di-($C_1$-$C_2$-alkyl)-amino steht,
Y für- Halogen, $C_1$-$C_2$-Alkyl, $C_1$-$C_2$-Halogenalkyl, Cyclopropyl, $C_1$-$C_2$-Alkoxy, $C_1$-$C_2$-Halogenalkoxy oder $C_1$-$C_2$-Alkylthio steht und
Z für Stickstoff oder eine CH-Gruppierung steht,
sowie Salze von Verbindungen der Formel (I).

2. Sulfonylaminoazine der allgemeinen Formel (I) gemäß Anspruch 1, dadurch gekennzeichnet, daß darin
$R^1$ für Fluor, Chlor, Brom, Iod, Cyano, Nitro, Methyl, Trifluormethyl, Chlordifluormethyl, Difluormethyl, Methoxy, Ethoxy, Difluormethoxy, Trifluormethoxy, Chlordifluormethoxy, 2-Chlor-ethoxy, 1,1,2,2-Tetrafluorethoxy, 2-Chlor-1,1,2-trifluorethoxy, Methylthio, Ethylthio, Difluormethylthio, Trifluormethylthio, Chlordifluormethylthio, Methylsulfinyl, Ethylsulfinyl, Trifluormethylsulfinyl, Methylsulfonyl, Ethylsulfonyl, Trifluormethylsulfonyl, 2-Methoxy-ethoxy, 2-Ethoxy-ethoxy, Dimethylaminosulfonyl, N-Methoxy-N-methylaminosulfonyl, Methoxycarbonyl oder Ethoxycarbonyl steht,
$R^2$ und $R^3$ unabhängig voneinander für Wasserstoff, Fluor, Chlor, Brom, Methyl oder Trifluormethyl stehen,
n für die Zahlen 0 oder 1 steht,
X für Wasserstoff, Methyl, Ethyl, Trifluormethyl, Chlormethyl, Methoxymethyl, Cyclopropyl, Methoxy, Ethoxy, Difluormethoxy, 2,2,2-Trifluorethoxy, Methoxymethoxy, 2-Methoxy-ethoxy, Methylthio, Methylamino, Ethylamino oder Dimethylamino steht,
Y für Fluor, Chlor, Brom, Methyl, Ethyl, Trifluormethyl, Chlormethyl, Cyclopropyl, Methoxy, Ethoxy, Difluormethoxy, 2,2,2-Trifluorethoxy oder Methylthio steht und
Z für Stickstoff oder eine CH-Gruppierung steht,
sowie die Natrium- und Kaliumsalze der Verbindungen der Formel (I).

3. Sulfonylaminoazine der allgemeinen Formel (1) gemäß Anspruch 1, dadurch gekennzeichnet, daß darin
$R^1$ für Fluor, Chlor, Brom, Methyl, Trifluormethyl, Difluormethoxy, Trifluormethoxy, 2-Chlor-ethoxy, Methylthio, Methylsulfonyl, Dimethylaminosulfonyl, Methoxycarbonyl oder Ethoxycarbonyl steht,
$R^2$ für Wasserstoff, Fluor oder Chlor steht, $R^3$ für Wasserstoff steht,
n für die Zahlen 0 oder 1 steht,
X für Methyl, Methoxy oder Ethoxy steht,
Y für Methyl, Methoxy oder Ethoxy steht und
Z für Stickstoff oder eine CH-Gruppierung steht.

4. Verfahren zur Herstellung von Sulfonylaminoazinen der allgemeinen Formel (I) gemäß Anspruch 1, dadurch gekennzeichnet, daß man

(a) Sulfonsäureamide der allgemeinen Formel (II)

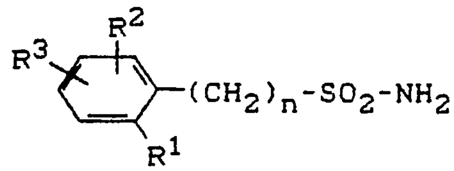

(II)

in welcher
$R^1$, $R^2$, $R^3$ und n die in Anspruch 1 angegebenen Bedeutungen haben,
mit Azinen der allgemeinen Formel (III)

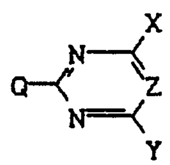

(III)

in welcher
X, Y und Z die in Anspruch 1 angegebenen Bedeutungen haben und
Q für eine nucleophile Abgangsgruppe steht,
gegebenenfalls in Gegenwart eines Säureakzeptors und gegebenenfalls in Gegenwart eines Verdünnungsmittels umsetzt, oder daß man

(b) Sulfonsäurechloride der allgemeinen Formel (IV)

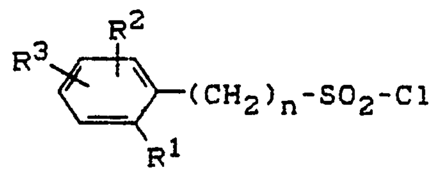

(IV)

in welcher
$R^1$, $R^2$, $R^3$ und n die in Anspruch 1 angegebenen Bedeutungen haben,
mit Aminoazinen der allgemeinen Formel (V)

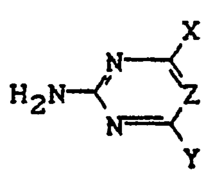

(V)

in welcher
X, Y und Z die in Anspruch 1 angegebenen Bedeutungen haben,

gegebenenfalls in Gegenwart eines Säureakzeptors und gegebenenfalls in Gegenwart eines Verdünnungsmittels umsetzt.

5. Herbizide Mittel, gekennzeichnet durch einen Gehalt an mindestens einem substituierten Sulfonylaminoazin der Formel (I) gemäß Anspruch 1.

6. Verfahren zur Bekämpfung von Unkräutern, dadurch gekennzeichnet, daß man substituierte Sulfonylaminoazine der Formel (I) gemäß Anspruch 1 auf die Unkräuter und/oder ihren Lebensraum einwirken läßt.

7. Verwendung von substituierten Sulfonylaminoazinen der Formel (I) gemäß Anspruch 1 zur Bekämpfung von Unkräutern.

8. Verfahren zur Herstellung von herbiziden Mitteln, dadurch gekennzeichnet, daß man substituierte Sulfonylaminoazine der Formel (I) gemäß Anspruch 1 mit Streckmitteln und/oder oberflächenaktiven Mitteln vermischt.